(19) Europäisches Patentamt
European Patent Office
Office européen des brevets

(11) Veröffentlichungsnummer: **0 099 538 B1**

(12) # EUROPÄISCHE PATENTSCHRIFT

(45) Veröffentlichungstag der Patentschrift: **11.09.91**

(51) Int. Cl.$^5$: **C07C 405/00**, C07C 59/62, C07C 69/734, A61K 31/557, //C07D317/72,C07D317/16

(21) Anmeldenummer: **83106792.1**

(22) Anmeldetag: **11.07.83**

Die Akte enthält technische Angaben, die nach dem Eingang der Anmeldung eingereicht wurden und die nicht in dieser Patentschrift enthalten sind.

(54) **Neue Carbacycline, Verfahren zu ihrer Herstellung und ihre Verwendung als Arzneimittel.**

(30) Priorität: **13.07.82 DE 3226550**

(43) Veröffentlichungstag der Anmeldung:
**01.02.84 Patentblatt 84/05**

(45) Bekanntmachung des Hinweises auf die Patenterteilung:
**11.09.91 Patentblatt 91/37**

(84) Benannte Vertragsstaaten:
**AT BE CH DE FR GB IT LI LU NL SE**

(56) Entgegenhaltungen:
**EP-A- 0 055 208**
**FR-A- 2 422 634**
**GB-A- 2 014 143**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Skuballa, Werner, Dr.**
**Olwenstrasse 25**
**W-1000 Berlin 28(DE)**
Erfinder: **Radüchel, Bernd, Dr.**
**Gollanczstrasse 132**
**W-1000 Berlin 28(DE)**
Erfinder: **Vorbrüggen, Helmut, Prof.**
**Wilkestrasse 7**
**W-1000 Berlin 27(DE)**
Erfinder: **Casals-Stenzel, Jorge, Dr.**
**Sertoriusring 295**
**W-6500 Mainz 21 (Finthen)(DE)**
Erfinder: **Mannesmann, (verehel.Varchmin), Gerda, Dr.**
**Sachsenring 22/24**
**W-5000 Köln 1(DE)**
Erfinder: **Schillinger, Ekkehard, Dr.**
**Im Amseltal 50**
**W-1000 Berlin 28(DE)**
Erfinder: **Town, Michael Harold, Dr.**
**Buchsbaumweg 3**
**W-1000 Berlin 47(DE)**

**EP 0 099 538 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Carbacyclin-Derivate, Verfahren zu ihrer Herstellung sowie ihre Verwendung als Arzneimittel. In den deutschen Offenlegungsschriften DE-OS 28 45 770, 29 00 352, 29 02 442, 29 04 655, 2909 088, 30 48 906 und 29 12 409 werden (5E)- und (5Z)-6a-Carbaprostaglandin-$I_2$-Analogabeschrieben. Die Nomenklatur der erfindungsgemäßen Verbindungen basiert auf einem Vorschlag von Morton und Brokaw (J. Org. Chem. 44, 2280 [1979]. Bei der Synthese dieser Verbindungen entstehen stets zwei Doppelbindungsisomere, die durch den Zusatz (5E) oder (5Z) charakterisiert werden. Die beiden Isomeren dieses Prototyps werden durch folgende Strukturformel verdeutlicht:

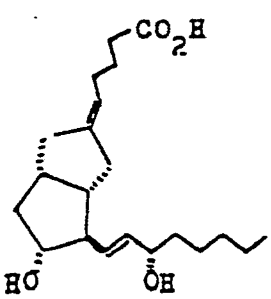

(5E)-6a-Carbaprostaglandin-$I_2$

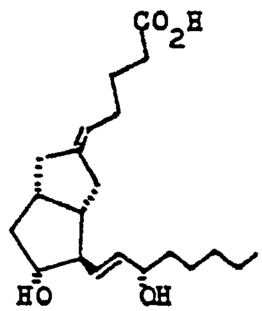

(5Z)-6a-Carbaprostaglandin-$I_2$

Aus dem sehr umfangreichen Stand der Technik der Prostacycline und ihrer Analoga weiß man, daß diese Stoffklasse aufgrund ihrer biolgoischen und pharmakologischen Eigenschaften zur Behandlung von Säugetieren, einschließlich Menschen geeignet ist. Ihre Verwendung als Arzneimittel stößt jedoch häufig auf Schwierigkeiten, da sie eine für therapeutische Zwecke zu kurze Wirkungsdauer besitzen.

Alle Strukturveränderungen haben das Ziel, die Wirkungsdauer sowie die Selektivität der Wirksamkeit zu steigern.

Es wurde nun gefunden, daß durch Homologisierung der oberen Kette der 3-Oxa-Carbacycline eine längere Wirkungsdauer, eine größere Selektivität und eine bessere Wirksamkeit erzielt werden kann. Die erfindungsgemäßen Verbindungen wirken blutdrucksenkend und bronchodilatorisch. Sie sind außerdem zur Vasodilatation, Inhibierung der Thrombocytenaggregation und der Magensäuresekretion geeignet.

Die Erfindung betrifft Carbacyclinderivate der allgemeinen Formel I

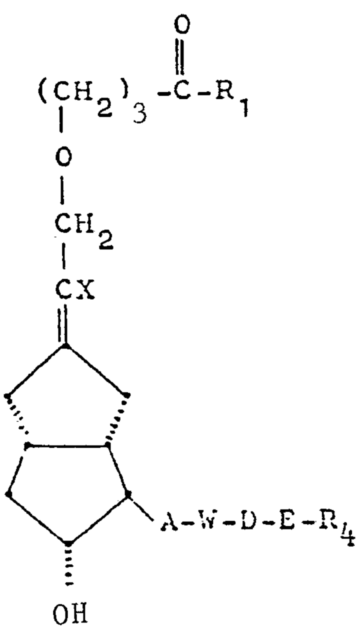

(I),

worin

- $R_1$ Wasserstoff oder OH,
- X ein Wasserstoffatom oder ein Fluoratom,
- A eines trans -CH=CH- oder -C≡C-Gruppe,
- W eine Hydroxymethylengruppe, wobei die OH-Gruppe α- oder β-standig sein kann,
- D die Gruppen

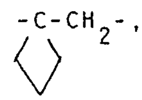

-$CH(CH_3)$-$CH_2$- oder -$C(CH_3)_2$-$CH_2$-,

- E eine -C≡C-Gruppe bedeuten,
- $R_4$ Methyl oder Ethyl und falls $R_1$ die Bedeutung einer OH-Gruppe hat, deren Salze mit physiologisch vertraglichen Basen bedeuten

Die Erfindung betrifft außerdem die nachstehenden Verbindungen:

(5E)-(16RS)-1a, 1b-Dihomo-16-methyl-3-oxa-18,18,19,19-tetradehydro-6a-carba-prostaglandin-$I_2$-methylester

(5E)-(16RS)-1a,1b-Dihomo-16-methyl-3-oxa-18,18,19,19-tetradehydro-6a-carba-prostaglandin-$I_2$-carboxamid

(5Z)-(16RS)-1a,Dihomo-5-fluor-16-methyl-3-oxa-18,18,19,19-tetradehydro-6a-carba-prostaglandin-$I_2$-(2,3-dihydroxy-propyl)-amid

(5Z)-(16RS)-1a,1b-Dihomo-5-fluor-16-methyl-3-oxa-18,18,19,19-tetradehydro-6a-carba-prostaglandin-$I_2$-(4-phenyl)-phenacylester

Zur Salzbildung mit den freien Säuren ($R_1$ = OH) sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der erfindungsgemäßen Carbacyclin-Derivate dar allgemeinen Formel I, dadurch gekennzeichnet, daß man in an sich bekannter Weise eine Verbindung der allgemeinen Formel II

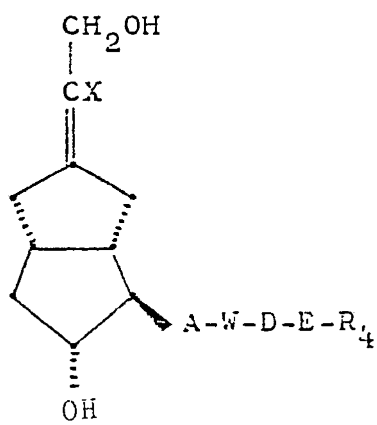

(II),

worin X, $R_4$, A, W, D und E die obenangegebenen Bedeutungen aufweisen, gegebenenfalls nach Schutz anwesender freier Hydroxygruppen mit einem Halogenketal der allgemeinen Formel III

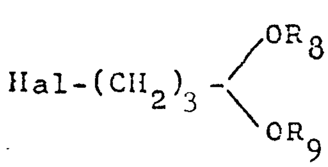

(III)

wobei Hal ein Chlor, Brom oder Jodatom, $R_8$ und $R_9$ eine Alkylgruppe mit 1 - 10 C-Atomen oder $R_8$ und $R_9$ gemeinsam eine ringbildende Gruppe mit 2 - 10 C-Atomen bedeuten, in Gegenwart einer Base veräthert und mit Säure das Ketal spaltet und gegebenenfalls anschließend in beliebiger Reihenfolge Isomere trennt und/oder geschützte Hydroxygruppen freisetzt und/oder freie Hydroxygruppen verestert oder veräthert und/oder die Aldehydgruppe oxidiert und/oder die resultierende freie Carboxylgruppe verestert und/oder eine veresterte Carboxylgruppe verseift oder eine Carboxylgruppe in ein Amid oder mit einer physiologisch verträglichen Base in ein Salz überführt.

Die Umsetzung der Verbindung der allgemeinen Formel II mit einem Halogenketal der allgemeinen Formel III wird bei Temperaturen von 0 °C bis 100 °C, vorzugsweise 10 °C bis 80 °C, in einem aprotischen Lösungsmittel oder Lösungsmittelgemisch, beispielsweise Dimethylsulfoxid, Dimethylformamid, Tetrahydrofuran usw., vorgenommen. Als Basen kommen die dem Fachmann für Verätherungen bekannten Basen in Frage, beispielsweise Natriumhydrid, Kalium-tert.-butylat, Butyllithium usw.

Die sich an die Verätherung anschließende Ketalspaltung zu den Verbindungen der allgemeinen Formel I erfolgt nach bekannten Methoden. Beispielsweise wird die Ketalspaltung in einer wäßrigen Lösung einer organischen Säure, wie zum Beispiel Essigsäure, Propionsäure u. a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Ketalspaltung wird vorzugsweise bei Temperaturen zwischen 20 °C und 80 °C durchgeführt.

Die Oxidation der Aldehydgruppe wird nach den dem Fachmann bekannten Methoden vorgenommen. Als Oxidationsmittel können beispielsweise dienen: Pyridiniumdichromat (Tetrahedron Letters, 1979, 399), Jones-Reagenz (J. Chem. Soc. 1953, 2555) oder Platin/Sauerstoff (Adv. in Carbohydrate Chem. 17, 169 (1962)).

Die Oxidation mit Pyridiniumdichromat wird bei Temperaturen von 0 °C bis 100 °C, vorzugweise 20 °C bis 40 °C, in einem geben das Oxidationsmittel inerten Lösungsmittel, beispielsweise Dimethylformamid, durchgeführt.

Die Oxidation mit Jones-Reagenz wird bei Temperaturen von -40 °C bis +40 °C, vorzugsweise 0 °C bis 30 °C, in Aceton als Lösungsmittel ausgeführt. Die Oxidation mit Platin/Sauerstoff wird bei Temperaturen von 0 °C bis 60 °C, vorzugsweise 20 °C bis 40 °C, in einen gegen das Oxidationsmittel inerten Lösungsmittel, wie zum Beispiel Essigester, durchgeführt.

Die Verseifung der Carbacyclinester wird nach den dem Fachmann bekannten Methoden durchgeführt, wie beispielsweise mit basischen Katalysatoren.

Die Einführung der Estergruppe mit $OCH_3$ für $R_1$, erfolgt nach den dem Fachmann bekannten Methoden. Die Carboxyverbindungen werden beispielsweise mit Diazomethan in an sich bekannter Weise umgesetzt. Die Veresterung mit Diazomethan erfolgt zum Beispiel dadurch, daß man eine Lösung des Diazomethans in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazomethan ist bekannt und kann nach bekannten Methodon hergestellt werden [Org. Reactions Bd. 8, Seiten 389 - 394 (1954)].

Die Einführung der Estergruppe mit

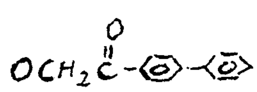

für $R_1$ kann auch durch Umsetzung des Carboxylatanions mit dem entsprechenden Alkylhalogenid oder ω-Halogenketon

$$(Hal\text{-}CH_2\text{-}\overset{O}{\overset{\|}{C}}\text{-}Ar,\ \text{mit Ar als Diphenyl}),$$

erfolgen.

Die Carbacyclin-Derivate der allgemeinen Formel I mit $R_1$ in der Bedeutung einer Hydroxygruppe

können mit geeigneten Mengen der entsprechenden anorganischen Basen unter Neutralisierung in Salze überführt werden. Beispielsweise erhält man beim Lösen der entsprechenden Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Die Herstellung der Aminsalze erfolgt in üblicher Weise. Dazu wird die Carbacyclinsäure zum Beispiel in einem geeigneten Lösungsmittel, wie Äthanol, Aceton, Diäthyläther oder Benzol, gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10fachen Menge des theoretischen Bedarfs. Die Umsetzung ist normalerweise bei 0 °C - 30 °C nach 15 bis 30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 °C und 80 °C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkalicarbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat.

Die Umsetzung erfolgt bei -10 °C bis 70 °C, vorzugsweise bei 25 °C. Die Einführung der Amidgruppe mit $NH_2$ oder $NHCH_2CH(OH)CH_2OH$ für $R_1$ erfolgt nach den dem Fachmann bekannten Methoden. Die Carbonsäuren der allgemeinen Formel I werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amins oder mit Ammoniak erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen -30 °C und +60 °C, vorzugsweise bei 0 °C bis 30 °C.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Äther- oder Acylreste intermediäre geschützt sind.

Die als Ausgangsmaterial dienenden Verbindungen der allgemeinen Formel II können beispielsweise hergestellt werden, indem man in an sich bekannter Weise einen Aldehyd der Formel V (DE-OS 28 45 770)

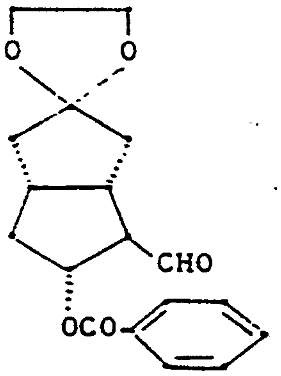

(V)

mit einem Phosphonat der allgemeinen Formel VI

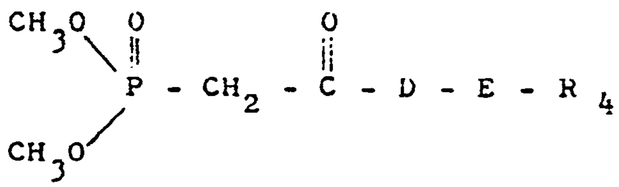

(VI)

worin D, E und $R_4$ die obenangegebene Bedeutung aufweisen, in Gegenwart eines Deprotonierungsmittels, wie beispielsweise Natriumhydrid oder Kalium-tert.-butylat, zu einem Keton der allgemeinen Formel VII (Z = H) oder zusätzlich in Gegenwart eines Bromierungsmittels, wie beispielsweise N-Bromsuccinimid, zu einem Keton der allgemeinen Formel VII (Z = Br) umsetzt.

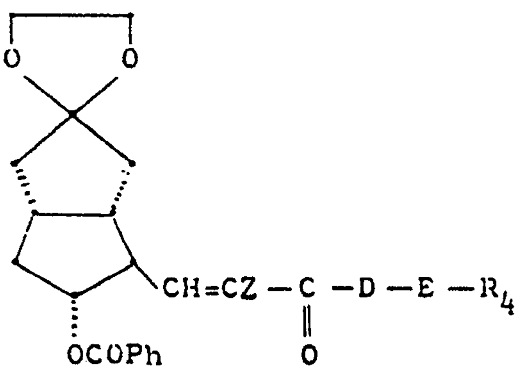

(VII)

Nach Reduktion der Ketogruppe mit Zinkborhydrid oder Natriumborhydrid oder Umsetzung mit Alkylmagnesiumbromid oder Alkyllithium und anschließender Epimerentrennung gelangt man zu den Verbindungen der allgemeinen Formel VIII

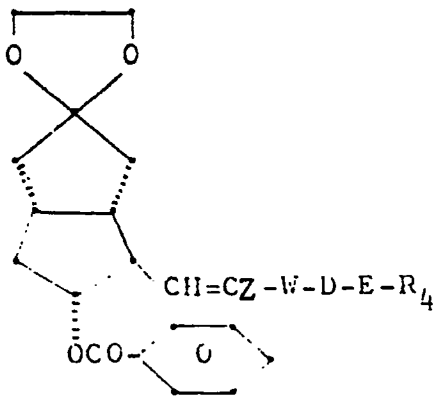

(VIII)

Verseifung der Estergruppe, beispielsweise mit Kaliumcarbonat in Methanol, sowie gegebenenfalls Hydrierung der Doppelbindung oder gegebenenfalls Verätherung mit Dihydropyran und anschließende Abspaltung von Bromwasserstoff mit beispielsweise Kalium-tert.-butylat in Dimethylsulfoxid, Ketalspaltung mit wäßriger Essigsäure sowie gegebenenfalls funktionelle Abwandlung der freien Hydroxygruppen, beispielsweise durch Verätherung mit Dihydropyran liefert das Keton der allgemeinen Formel IX.

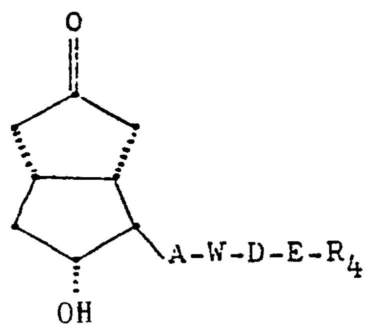

(IX)

Nach Olefinierungsreaktion mit Phosphonoessigsäuretriäthylester oder Phosphonoessigsäuretrimethylester oder Phosphonofluoressigsäuretriäthylester oder Phosphonofluoressigsäuretrimethylester und anschließender Reduktion mit Lithiumaluminiumhydrid erhält man die an der Doppelbindung isomeren Verbindungen der allgemeinen Formel II, die gegebenenfalls getrennt werden können.

Die Herstellung der Phosphonate der allgemeinen Formel VI erfolgt in an sich bekannter Weise durch Umsetzung des Anions von Methylphosphonsäuredimethylester mit einem Ester der allgemeinen Formel X

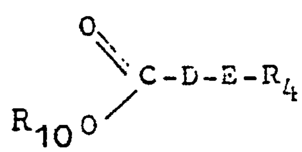

(X)

worin D, E, $R_4$ die obengenannten Bedeutungen aufweisen und $R_{10}$ eine Alkylgruppe mit 1 - 5 C-Atomen bedeutet, den man gegebenenfalls aus dem entsprechenden Malonsäureester durch Alkylierung mit dem Halogenid

$$Hal-E-R_4 \qquad (XI)$$

der allgemeinen Formel XI mit Hal in der Bedeutung Chlor oder Brom und anschließender Decarbalkoxylierung erhalten kann. Der Ester der allgemeinen Formel X ist gegebenenfalls auch aus der Carbonsäure der allgemeinen Formel XII

$$HO-\overset{\overset{\displaystyle O}{\|}}{C}-D \qquad (XII)$$

worin D die obenangegebene Bedeutung aufweist durch Alkylierung mit dem Halogenid der allgemeinen Formel XI und anschließender Veresterung zugänglich.

Die als Ausgangsmaterial dienenden Verbindungen der allgemeinen Formel III können beispielsweise hergestellt werden, indem man in an sich bekannter Weise einen ω-Halogencarbonsäureester der allgemeinen Formel XIII

$$Hal-(CH_2)_3-COOR_{10} \qquad (XIII)$$

worin Hal die obenangegebene Bedeutung aufweist und $R_{10}$ eine Alkylgruppe mit 1 - 5 C-Atomen bedeutet, mit Diisobutylaluminiumhydrid zum entsprechenden Aldehyd reduziert und anschließend in bekannter Weise mit einem. Alkohol ketalisiert.

Die Verbindungen dieser Erfindung wirken blutdrucksenkend und bronchodilatorisch. Sie sind weiterhin geeignet zur Hemmung der Thrombozyten-Aggregation. Folglich stellen die neuen Carbacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie zum Beispiel am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Analoga besitzen die für Prostacycline typischen Eigenschaften, wie zum Beispiel Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion, Zytoprotektion der Magen- und Darmschleimhaut, Zytoprotektion in der Leber und im Pankreas, antiallergische Eigenschaften, Senkung des pulmonaren vaskulären Widerstandes und des pulmonaren Blutdrucks, Förderung der Nierendurchblutung, Anwendung anstelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Carbacyclin-Derivate antiproliferative und antidiarrhoegene Eigenschaften. Die Carbacycline dieser Erfindung können auch in Kombination, zum Beispiel mit ß-Blockern oder Diuretika, verwendet werden.

Die Dosis der Verbindungen ist 1 - 1500 µg/kg/Tag, wenn sie am menschlichen Patienten verabreicht werden. Die Einheitsdosis für den pharmazeutischen akzeptablen Träger beträgt 0,01 - 100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen im Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wäßrige oder ölige Lösungen benutzt. Für die orale Applikation sind beispielsweise Tabletten, Dragées oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen, zum Beispiel zur Herstellung von Blutdrucksenkern, dienen.

Beispiel 1

(5E)-(16RS)-2-Descarboxy-1a,1b-dihomo-2-formyl-16-methyl-3-oxa-18,18,19,19-tetradehydro-6a-carba-prostaglandin-$I_2$

Zu einer Lösung von 700 mg 2-{(E)-(1S,5S,6R,7R)-7-(Tetrahydropyran-2-yloxy)-6-[(E)-(3S,4RS)-4-methyl-3-(tetrahydropyran-2-yloxy)-oct-1-en-6-in-yl]-bicyclo[3.3.0]octan-3-yliden}-äthan-1-ol in 15 ml Tetrahydrofuran fügt man bei 0° C 77 mg 55%ige Natriumhydridsuspension in Mineralöl und rührt 30 Minuten bei 24° C unter Argon. Anschließend tropft man eine Lösung von 1,15 g 2-(3-Brom-propyl)-1,3-dioxolan in 7 ml Tetrahydrofuran zu und kocht 21 Stunden am Rückfluß unter Argon. Man verdünnt mit Äther, wäscht mit Wasser neutral, trocknet über Magnesiumsulfat und dampft im Vakuum ein. Nach Chromatographie des Rückstandes an Kieselgel erhält man mit Hexan/Äther (7+3) 480 mg der Oxa-Verbindung, die man mit 40 ml einer Mischung aus Essigsäure/Wasser/Tetrahydrofuran (65+35+10) 16 Stunden bei 24° C rührt. Man dampft anschließend im Vakuum ein und chromatographiert den Rückstand an Kieselgel. Mit Essigester/Hexan (4+1) erhält man 270 mg der Titelverbindung als farbloses Öl.
IR ($CHCl_3$): 3600, 3420(breit), 2970, 2862, 2730, 1725, 1603, 970/cm
Das für die obige Verätherung verwendete 2-(3-Brom-propyl)-1,3-dioxolan wird wie folgt hergestellt:
Zu einer Lösung von 9,6 g Brombuttersäureäthylester in 595 ml Toluol tropft man langsam bei -70° C 50 ml einer 1,2 molaren Lösung von Diisobutylaluminiumhydrid in Toluol, rührt 15 Minuten bei -70° C und versetzt anschließend tropfenweise mit 10 ml Isopropylalkohol und 25 ml Wasser. Man rührt 2 Stunden bei Raumtemperatur, filtriert, trocknet das Filtrat mit Magnesiumsulfat und engt im Vakuum bei 25° C ein. Den Rückstand löst man in 500 ml Toluol, fügt 10 ml Äthylenglycol und 100 mg p-Toluolsulfonsäure zu und kocht 6 Stunden mit einem Wasserabscheider am Rückfluß. Anschließend verdünnt man mit 500 ml Äther, schüttelt einmal mit einer 5%igen Natriumbicarbonatlösung, dreimal mit Wasser, trocknet den organischen Extrakt mit Magnesiumsulfat und engt im Vakuum bei 30° C ein. Die Destillation des Rückstandes bei 0,6 Torr und 43°-45° C liefert 6,8 g 2-(3-Brompropyl)-1,3-dioxolan als farblose Flüssigkeit.

Beispiel 2

(5E)-(16RS)-1a,1b-Dihomo-16-methyl-3-oxa-18,18,19,19-tetradehydro-6a-carba-prostaglandin-$I_2$

Zu einer Lösung von 500 mg des nach Beispiel 1 hergestellten Aldehyds in 5 ml Pyridin fügt man 2 ml Essigsäurehydrid und läßt 18 Stunden bei Raumtemperatur stehen. Anschließend engt man im Vakuum ein, löst das erhaltene 11,15-Diacetat in 25 ml Aceton und versetzt bei 0° C tropfenweise mit 2,1 ml Jones Reagenz. Man rührt 30 Minuten bei 0° C, fügt 2 ml Isopropylalkohol zu, verdünnt mit Äther, schüttelt dreimal mit Wasser, trocknet über Magnesiumsulfat und dampft im Vakuum ein. Nach Chromatographie des Rückstandes an Kieselgel mit Hexan/Essigester (1+1) erhält man 410 mg (5E)-(16RS)-1a,1b-Dihomo-16-methyl-3-oxa-18,18,19,19-tetradehydro-6a-carba-prostaglandin-$I_2$-11,15-diacetat als farbloses Öl.
IR: 3650, 3400 (breit), 2960, 1730, 1600, 1245, 968/cm
Zur Abspaltung der Schutzgruppen rührt man 410 mg des 11,15-Diacetats in 20 ml Methanol 16 Stunden bei 24° C mit 520 mg Kaliumcarbonat. Anschließend konzentriert man im Vakuum, säuert mit 10%iger Citronensäurelösung auf pH4 an, extrahiert dreimal mit Methylenchlorid, wäscht zweimal mit Wasser, trocknet über Magnesiumsulfat und dampft im Vakuum ein. Den Rückstand chromatographiert man mit Essigester/Essigsäure (99,5+0,5) an Kieselgel. Dabei erhält man 305 mg der Titelverbindung als farbloses Öl.
IR: 3590, 342C (breit), 2960, 2930, 2865, 1720, 1600, 970/cm

Beispiel 3

(5Z)-(16RS)-2-Descarboxy-1a,1b-dihomo-2-formyl-16-methyl-3-oxa-18,18,19,19-tetradehydro-6a-carba-prostaglandin-$I_2$

In Analogie zu Beispiel 1 erhält man aus 320 mg 2-{(Z)-(1S,5S,6R,7R)-7-(Tetrahydropyran-2-yloxy)-6[-(E)-(3S,4RS)-4-methyl-3-(tetrahydropyran-2-yloxy)-oct-1-en-6-in-y1]-bicyclo[3.3.0]octan-3-yliden}-äthan-1-ol 125 mg der Titelverbindung als farbloses Öl.
IR: 3610, 3400 (breit), 2965, 2860, 2730, 1726, 1602, 968/cm

Beispiel 4

(5Z)-(16RS)-1a,1b-Dihomo-16-methyl-3-oxa-18,18,19,19-tetradehydro-6a-carba-prostaglandin-$I_2$

In Analogie zu Beispiel 2 erhält man aus 125 mg des nach Beispiel 3 hergestellten Aldehyds 90 mg (5Z)-(16RS)-1a,1b-Dihomo-16-methyl-3-oxa-18,18,19,19-tetradehydro-6a-carba-prostaglandin-$I_2$-11,15-diacetat. Nach Abspaltung der Schutzgruppen erhält man 57 mg der Titelverbindung als farbloses Öl.
IR: 3600, 3410 (breit), 2960, 2866, 1718, 1600, 968/cm

Beispiel 5

(5E)-(16R5)-2-Descarboxy-1a,1b-dihomo-16,20-dimethyl-2-formyl-3-oxa-18,18,19,19-tetradehydro-6a-carba-prostaglandin-$I_2$

In Analogie zu Beispiel 1 erhält man aus 1,35 g 2-{(E)-(1S,5S,6R,7R)-7-(Tetrahydropyran-2-yloxy)-6-[-(E)-(3S,4RS)-4-methyl-3-(tetrahydropyran-2-yloxy)-non-1-en-6-in-yl]-bicyclo[3.3.0]octan-3-yliden}-äthan-1-ol 610 mg der Titelverbindung als farbloses Öl.
IR: 3600, 3410 (breit), 2967, 2862, 2731, 1725, 1601, 970/cm
Das Ausgangsmaterial für die obige Titelverbindung wird wie folgt hergestellt:

5 a) (1R,5S,6R,7R)-3,3-Äthylendioxy-7-benzoyloxy-6-[(E)(3S,4RS)-3-hydroxy-4-methyl-non-1-en-6-in-yl]-bicyclo[3.3.0]octan

Zu einer Suspension von 1,46 g Natriumhydrid (55%ige Suspension in Öl) in 130 ml Dimethoxyäthan (DME) tropft man bei 0° C eine Lösung aus 9,02 g 3-Methyl-2-oxo-oct-5-inyl-phosphonsäure-dimethylester in 67 ml DME und rührt 1 Stunde bei 0° C. Anschließend versetzt man bei -20° C mit einer Lösung aus 9,4 g (1R,5S,6R,7R)-3,3-Äthylendioxy-7-benzoyloxy-6-formyl-bicyclo[3.3.0]-octan in 130 ml DME, rührt 1,5 Stunden bei -20° C, gießt auf 600 ml gesättigte Ammoniumchloridlösung und extrahiert dreimal mit Äther. Man wäscht den organischen Extrakt mit Wasser neutral, trocknet über Magnesiumsulfat und dampft im Vakuum ein. Nach Chromatographie des Rückstandes an Kieselgel erhält man mit Äther/Hexan (1+1) 9,1 g des $\alpha,\beta$-ungesättigten Ketons als Öl.
Zu einer Lösung von 9,1 g des Ketons in 300 ml Methanol fügt man bei -40° C portionsweise 5,2 g Natriumborhydrid und rührt 1 Stunde bei -40° C. Anschließend verdünnt man mit Äther, wäscht mit Wasser neutral, trocknet über Magnesiumsulfat und dampft im Vakuum ein. Durch Säulenchromatographie an Kieselgel mit Äther/Hexan erhält man zunächst 3,9 g der Titelverbindung (PG-Nomenklatur: 15$\alpha$-Hydroxy) sowie als polarere Komponente 3,2 g der isomeren 15$\beta$-Hydroxy-verbindung.
IR: 3600, 3400 (breit), 2942, 1711, 1603, 1588, 1276, 968, 947/cm

5 b) (1R,5S,6R,7R)-7-(Tetrahydropyran-2-yloxy)-6-[(E)-(3S,4RS)-3-(tetrahydropyran-2-yloxy)-4-methyl-non-1-en-6-in-yl]bicyclo [3.3.0]octan-3-on

Eine Mischung aus 3,6 g des nach Beispiel 5a hergestellten $\alpha$-Alkohols und 1,4 g Kaliumcarbonat in 120 ml Methanol rührt man 16 Stunden bei Raumtemperatur unter Argon. Anschließend engt man in Vakuum ein, verdünnt mit Äther und wäscht mit Sole neutral. Man trocknet über Magnesiumsulfat und dampft im Vakuum ein. Den Eindampfrückstand rührt man 16 Stunden bei Raumtemperatur mit 75 ml einer Mischung aus Essigsäure/Wasser/Tetrahydrofuran (65+35+10) und dampft anschließend im Vakuum ein. Nach Filtration des Rückstandes über Kieselgel erhält man mit Essigester/Hexan (7+3) 2,2 g des Ketons als Öl.
Eine Lösung aus 2,2 g des Ketons, 2,4 ml Dihydropyran und 23 mg p-Toluolsulfonsäure in 75 ml Methylenchlorid rührt man 30 Minuten bei 0° C. Anschließend verdünnt man mit Äther, schüttelt mit verd. Natriumbicarbonat-Lösung, wäscht mit Wasser neutral, trocknet über Magnesiumsulfat und dampft im Vakuum ein. Man erhält 3,4 g des Bis-tetrahydropyranyläthers, der ohne Reinigung verwendet wird.
IR: 2960, 2865, 1738, 970/cm

5 c) 2-{(E)-(1S,5S,6R,7R)-7-(Tetrahydropyran-2-yloxy-6-[(E)-(3S,4RS)-4-methyl-3-(tetrahydropyran-2-yloxy)-non-1-en-6-in-yl]bicyclo [3.3.0]octan-3-yliden}-äthan-1-ol

Zu einer Lösung von 8,1 g Phosphonessigsäuretriäthylester in 170 ml Tetrahydrofuran fügt man bei 0° C 3,5 g Kalium-tert.-butylat, rührt 10 Minuten, versetzt mit einer Lösung aus 9 g des nach Beispiel 5 b hergestellten Ketons in 90 ml Toluol und rührt 16 Stunden bei Raumtemperatur unter Argon. Man verdünnt mit 1000 ml Äther, schüttelt mit Wasser neutral, trocknet über Magnesiumsulfat und dampft im Vakuum ein. Den Rückstand filtriert man mit Hexan/Äther (3+2) über Kieselgel. Dabei erhält man 8,2 g des ungesättigten Esters als farbloses Öl.
IR: 2950, 2870, 1700, 1655, 968/cm
Man fügt 2,2 g Litiumaluminiumhydrid portionsweise bei 0° C zu einer gerührten Lösung von 8 g des vorstehend hergestellten Esters in 280 ml Äther und rührt 30 Minuten bei 0° C. Man zerstört den

Reagenzüberschuß durch tropfenweise Zugabe von Essigester, fügt 12 ml Wasser zu, rührt 2 Stunden bei 22° C, filtriert und dampft im Vakuum ein. Den Rückstand chromatographiert man mit Äther/Hexan (3+2) an Kieselgel. Dabei erhält man als unpolarere Verbindung 2,8 g 2-{(Z)-(1S,5S,6R,7R)-7-Tetrahydropyran-2-yloxy)-6[(E)-(3S,4RS)-4-methyl-3-(tetrahydropyran-2-yloxy)-non-1-en-6-in-yl]-bicyclo[3.3.0]-octan-3-yliden}-äthan-1-ol und 4,2 g der Titelverbindung als farbloses Öl.
IR: 3600, 3430, 2942, 2863, 1600, 972/cm

Beispiel 6

(5E)-(16RS)-1a,1b-Dihomo-16,20-dimethyl-3-oxa-18,18,19,19-tetradehydro-6a-carba-prostaglandin-$I_2$

In Analogie zu Beispiel 2 erhält man aus 380 mg des nach Beispiel 5 hergestellten Aldehyds 305 mg (5E)-(16RS)-1a,1b-Dihomo-16,20-dimethyl-3-oxa-18,18,19,19-tetradehydro-6a-carba-prostaglandin-$I_2$-11,15-diacetat.
Nach Abspaltung der Schutzgruppen erhält man 210 mg der Titelverbindung als farbloses Öl.
IR: 3600, 3400 (breit), 2962, 2865, 1720, 1601, 970/cm

Beispiel 7

(5E)-2-Descarboxy-1a,1b-dihomo-2-formyl-20-methyl-3-oxa-16,16-trimethylen-18,18,19,19-tetradehydro-6a-carba-prostaglandin-$I_2$

In Analogie zu Beispiel 1 und 5 erhält man aus 0,9 g 2-{(E)-(1S,5S, 6R,7R)-7-(Tetrahydropyran-2-yloxy)-6-[(E)-(3R)-3-(tetrahydropyran-2-yloxy)-4,4-trimethylen-non-1-en-6-in-yl]-bicyclo[3.3.0]octan-3-yliden}-äthan-1-ol (hergestellt gemäß Beispiel 5 a-c aus 2-Oxa-3,3-trimethylen-non-5-in-phosphonsäure-dimethylester) 0,4 g der Titelverbindung als farbloses Öl.
IR: 3610, 3400 (breit), 2968, 2864, 2730, 1725, 1602, 970/cm
Das Ausgangsmaterial für die obige Titelverbindung wird wie folgt hergestellt:

7 a) 2-{(E)-(1S,5S,6R,7R)-7-(Tetrahydropyran-2-yloxy)-4,4-trimethylen-non-1-en-6-in-yl]-bicyclo-[3.3.0]-octan-3-yliden}-äthan-1-ol

In Analogie zu Beispiel 5 c erhält man aus 3 g (1R,5S,6R,7R)-7-(Tetrahydropyran-2-yloxy)-6-[(E)-(3R)-3-(tetrahydropyran-2-yloxy)-4,4-trimethylen-non-1-en-6-inyl]-bicyclo[3.3.0]octan-3-on nach chromatographischer Isomerentrennung als unpolarere Verbindung 470 mg 2-{(Z)-(1S,5S,6R,7R)-7-(Tetrahydropyran-2-yloxy)-4,4-trimethylen-non-1-en-6-inyl]-bicyclo[3.3.0]octan-3-yliden}-äthan-1-ol und 690 mg der Titelverbindung als farbloses Öl.
IR: 3600, 3400 (breit), 2945, 2862, 1602, 972/cm

Beispiel 8

(5E)-1a,1b-Dihomo-20-methyl-3-oxa-16,16-trimethylen-18,18,19,19-tetradehydro-6a-carba-prostaglandin-$I_2$

In Analogie zu Beispiel 2 erhält man aus 400 mg des nach Beispiel 7 hergestellten Aldehyds 295 mg (5E)-1a,1b-Dihomo-20-methyl-3-oxa-16,16-trimethylen-18,18,19,19-tetradehydro-6a-carba-prostaglandin-$I_2$-11,15-diacetat.
Nach Abspaltung der Schutzgruppen erhält man 220 mg der Titelverbindung als farbloses Öl.
IR: 3610, 3400 (breit), 2960, 2864, 1721, 1602, 970/cm

Beispiel 9

(5E)-2-Descarboxy-1a,1b-dihomo-16,16-dimethyl-2-formyl-3-oxa-18,18,19,19-tetradehydro-6a-carba-prostaglandin-$I_2$

In Analogie zu Beispiel 1 und 5 erhält man 0,5 g 2-{(E)-(1S,5S,6R,7R)-7-(Tetrahydropyran-2-yloxy)-6-[-(E)-(3R)-4,4-dimethyl-3-(tetrahydropyran-2-yloxy)-oct-1-en-6-inyl]-bicyclo[3.3.0]octan-3-yliden}-äthan-1-ol 028 g der Titelverbindung als farbloses Öl.
IR: 3600, 3400 (breit), 2965, 2732, 1724, 1600, 970/cm

Beispiel 10

(5E)-1a,1b-Dihomo-16,16-dimethyl-3-oxa-18,18,19,19-tetradehydro-6a-carba-prostaglandin-$I_2$

In Analogie zu Beispiel 2 erhält man aus 0,27 g des nach Beispiel 9 hergestellten Aldehyds 180 mg (5E)-1a,1b-Dihomo-16,16-dimethyl-3-oxa-18,18,19,19-tetradehydro-6a-carba-prostaglandin-$I_2$-11,15-diacetat. Nach Abspaltung der Schutzgruppen erhält man 120 mg der Titelverbindung als farbloses Öl.
IR: 3600, 3400 (breit), 2962, 2865, 1720, 1600, 971/cm

Beispiel 11

(5E)-2-Descarboxy-1a,1b-dihomo-2-formyl-3-oxa-16,16,20-trimethyl-18,18,19,19-tetradehydro-6a-carba-prostaglandin-$I_2$

In Analogie zu Beispiel 1 und 5 erhält man aus 1,1 g 2-{(E)-(1S,5S, 6R,7R)-7-(Tetrahydropyran-2-yloxy)-6-[(E)-(3R)-4,4-dimethyl-3-(tetrahydropyran-2-yloxy)-non-1-en-6-inyl]-bicyclo[3.3.0]octan-3-yliden}-äthan-1-ol 0,6 g der Titelverbindung als farbloses Öl.
IR: 3610, 3420 (breit), 2964, 2730, 1725, 1602, 972/cm

Beispiel 12

(5E)-1a,1b-Dihomo-3-oxa-16,16,20-trimethyl-18,18,19,19-tetradehydro-6a-carba-prostaglandin-$I_2$

In Analogie zu Beispiel 2 erhält man aus 0,4 g des nach Beispiel 11 hergestellten Aldehyds 0,3 g (5E)-1a,1b-Dihomo-3-oxa-16,16-20-trimethyl-18,18,19,19-tetradehydro-6a-carba-prostaglandin-$I_2$-11,15-diacetat. Nach Abspaltung der Schutzgruppen erhält man 0,22 g der Titelverbindung als farbloses Öl.
IR: 3610, 3400 (breit), 2964, 2864, 1721, 1600, 972/cm

Beispiel 15

(5E)-(16RS)-2-Descarboxy-13,14-didehydro-1a,1b-dihomo-2-formyl-16-methyl-3-oxa-18,18,19,19-tetradehydro-6a-carba-prostaglandin-$I_2$

In Analogie zu Beispiel 1 und 5 erhält man aus 0,6 g 2-{(E)(1S,5S, 6S,7R)-7-(Tetrahydropyran-2-yloxy)-6-[(3S,4RS)-4-methyl-3-(tetrahydropyran-2-yloxy)-octa-1,6-diinyl]-bicyclo[3.3.0]octan-3-yliden]-äthan-1-ol 0,29 g der Titelverbindung als farbloses Öl.
IR: 3610, 3410 (breit), 2966, 2730, 2225, 1725/cm
Das Ausgangsmaterial für die obige Titelverbindung wird wie folgt hergestellt:

15 a) 2-{(E)-(1S,5S,6S,7R)-7-(Tetrahydropyran-2-yloxy)-6-[(3S,4RS)-4-methyl-3-(tetrahydropyran-2-yloxy)-octa-1,6-diinyl]-bicyclo [3.3.0]octan-3-yliden}-äthan-1-ol

In Analogie zu Beispiel 5 c erhält man aus 1,8 g (1R,5S,6S,7R)-7-(Tetrahydropyran-2-yloxy)-6-[-(3S,4RS)-4-methyl-3-tetrahydropyran-2-yloxy)-octa-1,6-diinyl]-bicyclo[3.3.0]octan-3-on nach chromatographischer Isomerentrennung als unpolarere Verbindung 380 mg 2-{(Z)-1S,5S,6R,7R)-7-(tetrahydropyran-2-yloxy)-6-[(3S,4RS)-4-methyl-3-(tetrahydropyran-2-yloxy)-octa-1,6-diinyl]-bicyclo[3.3.0]octan-3-yliden}-äthan-1-ol und 610 mg der Titelverbindung als Öl.
IR: 3600, 3400 (breit), 2945, 2860, 2225/cm

Beispiel 16

(5E)-(16RS)-13,14-Didehydro-1a,1b-dihomo-16-methyl-3-oxa-18,18,19,19-tetradehydro-6a-carba-prostaglandin-$I_2$

In Analogie zu Beispiel 2 erhält man aus 0,4 g des nach Beispiel 15 hergestellten Aldehyds 0,21 g (5E)-(16RS)-13,14-Didehydro-1a,1b-dihomo-16-methyl-3-oxa-18,18,19,19-tetradehydro-6a-carba-prostaglan din-$I_2$-11,15-diacetat.
Nach Abspaltung der Schutzgruppen erhält man 150 mg der Titelverbindung als farbloses Öl.
IR: 3600, 3410 (breit), 2960, 2864, 2226, 1718/cm

Beispiel 17

(5E)-(16RS)-2-Descarboxy-13,14-didehydro-1a,1b-dihomo-16,20-dimethyl-2-formyl-3-oxa-18,18,19,19-tetradehydro-6a- carba-prostaglandin-$I_2$

In Analogie zu Beispiel 1 und 5 erhält man aus 0,8 g 2-{(E)-(1S,5S, 6S,7R)-7-(Tetrahydropyran-2-yloxy)-6-[(3S,4RS)-4-methyl-3-(tetrahydropyran-2-yloxy)-nona-1,6-diinyl]-bicyclo[3.3.0]octan-3-yliden}-äthan-1-ol 0,42 g der Titelverbindung als farbloses Öl.
IR: 3600, 3400 (breit), 2965, 2732, 2227, 1724/cm
Das Ausgangsmaterial für die obige Titelverbindung wird wie folgt hergestellt:

17 a) 2-{(E)-(1S,5S,6S,7R)-7-(Tetrahydropyran-2-yloxy)-6-(3S,4RS)-4-methyl-3-(tetrahydropyran-2-yloxy)-nona-1,6-diinyl]-bicyclo [3.3.0]octan-3-yliden}-äthan-1-ol

In Analogie zu Beispiel 5 c erhält man aus 2,1 g (1R,5S,6S,7R)-7-(Tetrahydropyran-2-yloxy)-6-[-(3S,4RS)-4-methyl-3-(tetrahydropyran-2-yloxy)-nona-1,6-diinyl]bicyclo[3.3.0]octan-3-on nach chromatographischer Isomerentrennung als unpolarere Verbindung 450 mg 2-{(Z)-(1S,5S,6S,7R)-7-(Tetrahydropyran-2-yloxy)-6-[(3S,4RS)-4-methyl-3-(tetrahydropyran-2-yloxy)-nona-1,6-diinyl]-bicyclo-[3.3.0]octan-3-yliden}-äthan-1-ol und 740 mg der Titelverbindung als farbloses Öl.
IR: 3600, 3420 (breit), 2947, 2862, 2223/cm

Beispiel 18

(5E)-(16RS)-13,14-Didehydro-1a,1b-dihomo-16,20-dimethyl-3-oxa-18,18,19,19-tetradehydro-6a-carba-prostaglandin-$I_2$

In Analogie zu Beispiel 2 erhält man aus 620 mg des nach Beispiel 17 hergestellten Aldehyds 340 mg (5E)-(16RS)-13,14-Didehydro-1a,1b-dihomo-16,20-dimethyl-3-oxa-18,18,19,19-tetradehydro-6a-carba-prostaglandin-$I_2$-11,15-diacetat.
Nach Abspaltung der Schutzgruppen erhält man 260 mg der Titelverbindung als farbloses Öl.
IR: 3610, 3400 (breit), 2962, 2865, 2225, 1720/cm

Beispiel 19

(5E)-2-Descarboxy-13,14-didehydro-1a,1b-dihomo-2-formyl-2C-methyl-3-oxa-18,18,19,19-tetradehydro-16,16-trimethylen-6a-carba-prostaglandin-$I_2$

In Analogie zu Beispiel 1 und 5 erhält man aus 0,41 g 2-{(E)-(1S,5S, 6S,7R)-7-(Tetrahydropyran-2-yloxy)-6-[(3S)-(tetrahydropyran-2-yloxy)-4,4-trimethylen-nona-1,6-diinyl]-bicyclo[3.3.0]octan-3-yliden}-äthan-1-ol 0,18 g der Titelverbindung als farbloses Öl.
IR: 3600, 3400 (breit), 2965, 2732, 2227, 1724/cm
Das Ausgangsmaterial für die obige Titelverbindung wird wie folgt hergestellt:

19 a) 2-{(E)-(1S,5S,6S,7R)-7-(Tetrahydropyran-2-yloxy)-6-[(3S)-3-(tetrahydropyran-2-yloxy)-4,4-trimethylen-nona-1,6-diinyl]-bicyclo[3.3.0]octan-3-yliden}-äthan-1-ol

In Analogie zu Beispiel 5 c erhält man aus 3,1 g (1R,5S,6S,7R)-7-(Tetrahydropyran-2-yloxy)-6-[(3S)-3-(tetrahydropyran-2-yloxy)-4,4-trimethylen-nona-1,6-diinyl]-bicyclo[3.3.0]octan-3-on nach chromatographischer Isomerentrennung als unpolarere Verbindung 890 mg 2-{(Z)-(1S,5S,6S,7R)-7-(Tetrahydropyran-2-yloxy)-6-[(3RS)-3-(tetrahydropyran-2-yloxy)-4,4-trimethylen-nona-1,6-diinyl]-bicyclo[3.3.0]octan-3-yliden}-äthan-1-ol und 1,3 g der Titelverbindung als Öl.
IR: 3610, 3420 (breit), 2945, 2862, 2226/cm

Beispiel 20

(5E)-13,14-Didehydro-1a,1b-dihomo-20-methyl-3-oxa-18,18,19,19-tetradehydro-16,16-trimethylen-6a-carba-prostaglandin-$I_2$

In Analogie zu Beispiel 2 erhält man aus 0,42 g des nach Beispiel 19 hergestellten Aldehyds 0,32 g (5E)-13,14-Didehydro-1a,1b-dihomo-20-methyl-3-oxa-18,18,19,19-tetradehydro-16,16-trimethylen-6a-carba-prostaglandin-$I_2$-11,15-diacetat.
Nach Abspaltung der Schutzgruppen erhält man 210 mg der Titelverbindung als farbloses Öl.
IR: 3600, 3400 (breit), 2963, 2865, 2225, 1720/cm

Beispiel 21

(5E)-2-Descarboxy-13,14-didehydro-1a,1b-dihomo-16,16-dimethyl-2-formyl-3-oxa-18,18,19,19-tetradehydro-6a-carba-prostaglandin-$I_2$

In Analogie zu Beispiel 1 und 5 erhält man aus 0,9 g 2-{(E)-(1S,5S, 6S,7R)-7-(Tetrahydropyran-2-yloxy)-6-[(3S)-4,4-dimethyl-3-(tetrahydropyran-2-yloxy)-octa-1,6-diinyl]-bicyclo[3.3.0]octan-3-yliden}-äthan-1-ol 0,47 g der Titelverbindung als farbloses Öl.
IR: 3600, 3410 (breit), 2966, 2730, 2225, 1725/cm
Das Ausgangsmaterial für die obige Titelverbindung wird wie folgt hergestellt:

21 a) 2-{(E)-(1S,5S,6S,7R)-7-(Tetrahydropyran-2-yloxy)-6-[(3S)-4,4-dimethyl-3-(tetrahydropyran-2-yloxy)-octa-1,6-diinyl]-bicyclo [3.3.0]octan-3-yliden}-äthan-1-ol

In Analogie Zu Beispiel 5 c erhält man aus 2,5 g (1R,5S,6S,7R)-7-(Tetrahydropyran-2-yloxy)-6-[(3S)-4,4-dimethyl-3-(tetrahydropyran-2-yloxy)-octa-1,6-diinyl]-bicyclo[3.3.0]octan-3-on nach chromatographischer Isomerentrennung als unpolarere Verbindung 625 mg 2-{(Z)-(1S,5S,6S,7R)-7-(Tetrahydropyran-2-yloxy)-6-[(3S)-4,4-dimethyl-3-(tetrahydropyran-2-yloxy)-octa-1,6-diinyl]-bicyclo [3.3.0]octan-3-yliden}-äthan-1-ol und 1,1 g der Titelverbindung als Öl.
IR: 3600, 3400 (breit), 2946, 2865, 2225/cm

Beispiel 22

(5E)-13,14-Didehydro-1a,1b-dihomo-16,16-dimethyl-3-oxa-18,18,19,19-tetradehydro-6a-carba-prostaglandin-$I_2$

In Analogie zu Beispiel 2 erhält man aus 0,31 g des nach Beispiel 21 hergestellten Aldehyds 0,21 g (5E)-13,14-Didehydro-1a,1b-dihomo-16,16-dimethyl-3-oxa-18,18,19,19-tetradehydro-6a-carba-prostaglandin-$I_2$-11,15-diacetat.
Nach Abspaltung der Schutzgruppen erhält man 0,14 g der Titelverbindung als farbloses Öl.
IR: 3600, 3410 (breit), 2964, 2865, 2225, 1720/cm

Beispiel 23

(5E)-2-Descarboxy-13,14-didehydro-1a,1b-dihomo-2-formyl-3-oxa-18,18,19,19-tetradehydro-16,16,20-trimethyl-6a-carba-prostaglandin-$I_2$

In Analogie Zu Beispiel 1 und 5 erhält man aus 0,8 g 2-{(E)-(1S,5S,6S,7R)-7-(Tetrahydropyran-2-yloxy)-6-[(3S)-4,4-dimethyl-3-(tetrahydropyran-2-yloxy)-nona-1,6-diinyl]-bicyclo[3.3.0]octan-3-yliden}-äthan-1-ol 0,31 g der Titelverbindung als farbloses Öl.
IR: 3610, 3420 (breit), 2965, 2730, 2226, 1724/cm
Das Ausgangsmaterial für die obige Titelverbindung wird wie folgt hergestellt:

23 a) 2-{(E)-(1S,5S,6S,7R)-7-(Tetrahydropyran-2-yloxy)-6-[(3S)-4,4-dimethyl-3-(tetrahydropyran-2-yloxy)-nona-1,6-diinyl]-bicyclo [3.3.0]octan-3-yliden}-äthan-1-ol

In Analogie zu Beispiel 5 c erhält man aus 1,3 g (1R,5S,6S,7R)-7-(Tetrahydropyran-2-yloxy)6-[(3S)-4,4-dimethyl-3-(tetrahydropyran-2-yloxy)-nona-1,6-diinyl]-bicyclo[3.3.0]octan-3-on nach chromatographischer Isomerentrennung als unpolarere Verbindung 300 mg 2-{(Z)-(1S,5S,6S,7R)-7-(Tetrahydropyran-2-yloxy)-6-[(3S)-4,4-dimethyl-3-(tetrahydropyran-2-yloxy)-nona-1,6-diinyl]-bicyclo[3.3.0]octan-3-yliden}-äthan-1-ol und 430 mg der Titelverbindung als Öl.
IR: 3610, 3400 (breit), 2945, 2865, 2225/cm

Beispiel 24

(5E)-13,14-Didehydro-1a,1b-dihomo-3-oxa-18,18,19,19-tetradehydro-16,16,20-trimethyl-6a-carba-prostaglandin-$I_2$

In Analogie zu Beispiel 2 erhält man aus 0,16 g des nach Beispiel 23 hergestellten Aldehyds 0,1 g (5E)-13,14-Didehydro-1a,1b-dihomo-3-oxa-18,18,19,19-tetradehydro-16,16,20-trimethyl-6a-carba-prostaglandin-$I_2$-11,15-diacetat.
Nach Abspaltung der Schutzgruppen erhält man 60 mg der Titelverbindung als farbloses Öl.

IR: 3600, 3400 (breit), 2965, 2864, 2224, 1718/cm

Beispiel 25

(5Z)-(16RS)-2-Descarboxy-1a,1b-dihomo-5-fluor-2-formyl-16-methyl-3-oxa-18,18,19,19-tetradehydro-6a-carba-prostaglandin-$I_2$

Zu einer Lösung von 420 mg 2-{(Z)-(1S,5S,6R,7R)-7-(Tetrahydropyran-2-yloxy)-6-[(E)-(3S,4RS)-4-methyl-3-(tetrahydropyran-2-yloxy)-oct-1-en-6-inyl]-bicyclo-[3.3.0]octan-3-yliden}-2-fluor-äthan-1-ol in 8 ml Tetrahydrofuran fügt man bei 0° C 42 mg 55%ige Natriumhydridsuspension in Mineralöl und rührt 30 Minuten bei 24° C unter Argon. Anschließend tropft man eine Lösung von 630 mg 2-(3-Brompropyl)-1,3-dioxolan in 8 ml Tetrahydrofuran zu und kocht 20 Stunden am Rückfluß unter Argon. Man verdünnt mit Äther, wäscht mit Wasser neutral, trocknet über Magnesiumsulfat und dampft im Vakuum ein. Nach Chormatographie des Rückstandes an Kieselgel erhält man mit Hexan/Äther (3+2) 340 mg der Oxa-Verbindung, die man mit 30 ml einer Mischung aus Essigsäure/Wasser/Tetrahydrofuran (65+35+10) 16 Stunden bei 24° C rührt. Man dampft anschließend im Vakuum ein und chromatographiert den Rückstand an Kieselgel. Mit Essigester/Hexan (4+1) erhält man 280 mg der Titelverbindung als farbloses Öl.
IR: 3600, 3420 (breit), 2960, 2930, 2370, 2730, 1730, 1603, 970/cm

Beispiel 26

(5Z)-(16RS)-1a,1b-Dihomo-5-fluor-16-methyl-3-oxa-18,18,19,19-tetradehydro-6a-carba-prostaglandin-$I_2$

In Analogie zu Beispiel 2 erhält man aus 205 mg des nach Beispiel 25 hergestellten Aldehyds 110 mg (5Z)-(16RS)-1a,1b-Dihomo-5-fluor-16-methyl-3-oxa-18,18,19,19-tetradehydro-6a-carba-prostaglandin-$I_2$-11, 15-diacetat.
Nach Abspaltung der Schutzgruppen erhält man 78 mg der Titelverbindung als farbloses Öl.

Beispiel 27

(5Z)-(16RS)-2-Descarboxy-1a,1b-dihomo-16,20-dimethyl-5-fluor-2-formyl-3-oxa-18,18,19,19-tetradehydrc-6a-carba-prostaglandin-$I_2$

In Analogie zu Beispiel 25 erhält man aus 610 mg 2-{(E)-(1S,5S,6R,7R)-7-(Tetrahydropyran-2-yloxy)-6-[-(3S,4RS)-4-methyl-3-(tetrahydropyran-2-yloxy)-non-1-en-6-inyl]-bicyclo[3.3.0]octan-3-yliden}-2-fluor-äthan-1-ol 370 mg der Titelverbindung als farbloses Öl.
IR: 3610, 3400 (breit), 2963, 2930, 2868, 2731, 1630, 1602, 971/cm

Beispiel 28

(5Z)-(16RS)-1a,1b-Dihomo-16,20-dimethyl-5-fluor-3-oxa-18,18,19,19-tetradehydro-6a-carba-prostaglandin-$I_2$

In Analogie zu Beispiel 2 erhält man aus 230 mg des nach Beispiel 27 hergestellten Aldehyds 125 mg (5Z)-(16RS)-1a,1b-Dihomo-16,20-dimethyl-5-fluor-3-oxa-18,18,19,19-tetradehydro-6a-carba-prostaglandin-$I_2$-11,15-diacetat.
Nach Abspaltung der Schutzgruppen erhält man 85 mg der Titelverbindung als farbloses Öl.
IR: 3600, 3410 (breit), 2965, 2930, 2870, 1720, 1602, 970/cm

Beispiel 29

(5Z)-2-Descarboxy-1a,1b-dihomo-5-fluor-2-formyl-20-methyl-3-oxa-18,18,19,19-tetradehydro-16,16-trimethylen-6a-carba-prostaglandin-$I_2$

In Analoge zu Beispiel 25 erhält man aus 390 mg 2-{(Z)-(1S,5S,6R,7R)-7-(Tetrahydropyran-2-yloxy)-6-[-(E)-(3R)-3-(tetrahydropyran-2-yloxy)-4,4-trimethylen-non-1-en-6-inyl]-bicyclo[3.3.0]octan-3-yliden}-2-fluor-äthan-1-ol 165 mg der Titelverbindung als farbloses Öl.
IR: 3600, 3410 (breit), 2965, 2931, 2870, 2730, 1630, 1601, 970/cm

Beispiel 30

(5Z)-1a,1b-Dihomo-5-fluor-20-methyl-3-oxa-18,18,19,19-tetradehydro-16,16-trimethylen-6a-carba-prostaglandin-$I_2$

In Analogie zu Beispiel 2 erhält man aus 190 mg des nach Beispiel 29 hergestellten Aldehyds 105 mg (5Z)-1a,1b-Dihomo-5-fluor-20-methyl-3-oxa-18,18,19,19-tetradehydro-16,16-trimethylen-6a-carba-prostaglandin-$I_2$-11,15-diacetat.
Nach Abspaltung der Schutzgruppen erhält man 70 mg der Titelverbindung als fabrloses Öl.
IR: 3600, 3400 (breit), 2965, 2930, 2870, 1718, 1602, 970/cm

Beispiel 31

(5Z)-2-Descarboxy-1a,1b-dihomo-16,16-dimethyl-5-flour-2-formyl-3-oxa-18,18,19,19-tetradehydro-6a-carba-prostaglandin-$I_2$

In Analogie zu Beispiel 25 erhält man aus 0,6 g 2-{(Z)-(1S,5S,6R,7R)-7-(Tetrahydropyran-2-yloxy)-6-[-(E)-(3R)-4,4-dimethyl-3-(tetrahydropyran-2-yloxy)-oct-1-en-6-inyl]-bicyclo[3.3.0]octan-3-yliden}-2-fluoräthan-1-ol 0,27 g der Titelverbindung als farbloses Öl.
IR: 3610, 3420 (breit), 2966, 2930, 2868, 2732, 1730, 1602, 971/cm

Beispiel 32

(5Z)-1a,1b-Dihomo-16,16-dimethyl-5-fluor-3-oxa-18,18,19,19-tetradehydro-6a-carba-prostaglandin-$I_2$

In Analogie zu Beispiel 2 erhält man aus 220 mg des nach Beispiel 31 hergestellten Aldehyds 120 mg (5Z)-1a,1b-Dihomo-16,16-dimethyl-5 fluor-3-oxa-18,18,19,19-tetradehydro-6a-carba-prostaglandin-$I_2$-11, 15-diacetat.
Nach Abspaltung der Schutzgruppen erhält man 92 mg der Titelverbindung als farbloses Öl.
IR: 3600, 3410 (breit), 2964, 2931, 2870, 1720, 1601, 971/cm

Beispiel 33

(5Z)-2-Descarboxy-1a,1b-dihomo-5-fluor-2-formyl-3-oxa-18,18,19,19-tetradehydro-16,16,20-trimethyl-6a-carba-prostaglandin-$I_2$

In Analogie zu Beispiel 25 erhält man aus 0,7 g 2-{(Z)-(1S,5S,6R,7R)-7-(Tetrahydropyran-2-yloxy)-6-[-(E)-(3R)-4,4-dimethyl-3-(tetrahydropyran-2-yloxy)-non-1-en-6-inyl]-bicyclo[3.3.0]octan-3-yliden}-2-fluoräthan-1-ol 0,38 g der Titelverbindung als farbloses Öl.
IR: 3610, 3400 (breit), 2965, 2930, 2870, 2730, 1730, 1601, 970/cm

Beispiel 34

(5Z)-1a,1b-Dihomo-5-fluor-3-oxa-18,18,19,19-tetradehydro-16,16,20-trimethyl-6a-carba-prostaglandin-$I_2$

In Analogie zu Beispiel 2 erhält man aus 0,3 g des nach Beispiel 33 hergestellten Aldehyds 0,16 g (5Z)-1a,1b-Dihomo-5-fluor-3-oxa-18,18, 19,19-tetradehydro-16,16,20-trimethyl-6a-carba-prostaglandin-$I_2$-11,15-diacetat.
Nach Abspaltung der Schutzgruppen erhält man 0,12 g der Titelverbindung als farbloses Öl.
IR: 3610, 3400 (breit), 2965, 2868, 1720, 1602, 971/cm

Beispiel 35

(5Z)-(16RS)-2-Descarboxy-13,14-didehydro-1a,1b-dihomo-5-fluor-2-formyl-16-methyl-3-oxa-18,18,19,19-tetradehydro-6a-carba-prostaglandin-$I_2$

In Analogie zu Beispiel 25 erhält man aus 0,41 g 2-{(Z)-(1S,5S,6S,7R)-7-(Tetrahydropyran-2-yloxy)-6[-(3S,4RS)-4-methyl-3-(tetrahydropyran-2-yloxy)-ocat-1,6-diinyl]-bicyclo[3.3.0]octan-3-yliden}-5-fluor-äthan-1-ol 0,2 g der Titelverbindung als farbloses Öl.
IR: 3600, 3410 (breit), 2966, 2731, 2224, 1727/cm

Beispiel 36

(5Z)-(16RS)-13,14-Didehydro-1a,1b-dihomo-5-fluor-16-methyl-3-oxa-18,18,19,19-tetradehydro-6a-carba-prostaglandin-$I_2$

In Analogie zu Beispiel 2 erhält man aus 0,2 g des nach Beispiel 35 hergestellten Aldehyds 0,1 g (5Z)-(16RS)-13,14-Didehydro-1a,1b-dihomo-5-fluor-16-methyl-3-oxa-18,18,19,19-tetradehydro-6a-carba-prostaglandin-$I_2$-11,15-diacetat.
Nach Abspaltung der Schutzgruppen erhält man 70 mg der Titelverbindung als farbloses Öl.
IR: 3620, 3400 (breit), 2965, 2870, 2225, 1620/cm

Beispiel 37

(5Z)-(16RS)-2-Descarboxy-13,14-didehydro-1a,1b-dihomo-16,20-dimethyl-5-fluor-2-formyl-3-oxa-18,18,19,19-tetradehydro-6a-carba-prostaglandin-$I_2$

In Analogie zu Beispiel 25 erhält man aus 0,7 g 2-{(Z)-(1S,5S,6S,7R)-7-(Tetrahydropyran-2-yloxy)-6-[-(3S,4RS)-4-methyl-3-(tetrahydropyran-2-yloxy)-nona-1,6-diinyl]-bicyclo[3.3.0]octan-3-yliden}-2-fluor-äthan-1-ol 0,38 g der Titelverbindung als farbloses Öl.
IR: 3600, 3400 (breit), 2968, 2730, 2225, 1728/cm

Beispiel 38

(5Z)-(16RS)-13,14-Didehydro-1a,1b-dihomo-16,20-dimethyl-5-fluor-3-oxa-18,18,19,19-tetradehydro-6a-carba-prostaglandin-$I_2$

In Analogie zu Beispiel 2 erhält man aus 0,35 g des nach Beispiel 37 hergestellten Aldehyds 0,18 g (5Z)-(16RS)-13,14-Didehydro-1a,1b-dihomo-16,20-dimethyl-5-fluor-3-oxa-18,18,19,19-tetradehydro-6a-carba-prostaglandin-$I_2$-11,15-diacetat.
Nach Abspaltung der Schutzgruppen erhält man 0,14 g der Titelverbindung als farbloses Öl.
IR: 3600, 3420 (breit), 2966, 2870, 2226, 1718/cm

Beispiel 39

(5Z)-2-Descarboxy-13,14-didehydro-1a,1b-dihomo-5-fluor-2-formyl-20-methyl-3-oxa-18,18,19,19-tetradehydro-16,16-trimethylen-6a-carba-prostaglandin-$I_2$

In Analogie zu Beispiel 25 erhält man aus 1,2 g 2-{(Z)-(1S,5S,6S,7R)-7-(Tetrahydropyran-2-yloxy)-6-[-(35)-3-(tetrahydropyran-2-yloxy)-4,4-trimethylen-nona-1,6-diinyl]-bicyclo[3.3.0]octan-3-yliden}-2-fluoräthan-1-ol 0,7 g der Titelverbindung als farbloses Öl.
IR: 3620, 3420 (breit), 2970, 2731, 2224, 1730/cm

Beispiel 40

(5Z)-13,14-Didehydro-1a,1b-dihomo-5-fluor-20-methyl-3-oxa-18,18,19,19-tetradehydro-16,16-trimethylen-6a-carba-prostaglandin-$I_2$

In Analogie zu Beispiel 2 erhält man aus 0,6 g des nach Beispiel 39 hergestellten Aldehyds 0,31 g (5Z)-13,14-Didehydro-1a,1b-dihomo-5-fluor-20-methyl-3-oxa-18,18,19,19-tetradehydro-16,16-trimethylen-6a-carba-prostaglandin-$I_2$-11,15-diacetat.
Nach Abspaltung der Schutzgruppen erhält man 0,25 g der Titelverbindung als farbloses Öl.
IR: 3620, 3425 (breit), 2968, 2870, 2225, 1720/cm

Beispiel 41

(5Z)-2-Descarboxy-13,14-didehydro-1a,1b-dihomo-16,16-dimethyl-5-fluor-2-formyl-3-oxa-18,18,19,19-tetradehydro-6a-carba-prostaglandin-$I_2$

In Analogie zu Beispiel 25 erhält man aus 1,4 g 2-{(Z)(1S,5S,6S,7R)-7-(Tetrahydropyran-2-yloxy)-6-[-(3S)-4,4-dimethyl-3-(tetrahydropyran-2-yloxy)octa-1,6-diinyl]-bicyclo[3.3.0]octan-3-yliden}-2-fluor-äthan-1-ol 0,65 g der Titelverbindung als farbloses Öl.
IR: 3600, 3420 (breit), 2970, 2930, 2865, 2730, 2225, 1730/cm

Beispiel 42

(5Z)-13,14-Didehydro-1a,1b-dihomo-16,16-dimethyl-5-fluor-3-oxa-18,18, 19,19-tetradehydro-6a-carba-prostaglandin-$I_2$

In Analogie zu Beispiel 2 erhält man aus 0,4 g des nach Beispiel 41 hergestellten Aldehyds 0,22 g (5Z)-13,14-Didehydro-1a,1b-dihomo-16,16-dimethyl-5-fluor-3-oxa-18,18,19,19-tetradehydro-6a-carba-prostaglandin-$I_2$-11,15 diacetat.
Nach Abspaltung der Schutzgruppen erhält man 0,18 g der Titelverbindung als farbloses Öl.
IR: 3600, 3420 (breit), 2970, 2870, 2224, 1718/cm

Beispiel 43

(5Z)-2-Descarboxy-13,14-didehydro-1a,1b-dihomo-5-fluor-2-formyl-3-oxa-18,18,19,19-tetradehydro-16,16,20-trimethyl-6a-carba-prostaglandin-$I_2$

In Analogie zu Beispiel 25 erhält man aus 0,7 g 2-{(Z)- (1S,5S,6S,7R)-7-(Tetrahydropyran-2-yloxy)-6-[-(3S)-4,4-dimethyl-3-(tetrahydropyran-2-yloxy)-nona-1,6-diinyl]-bicyclo[3.3.0]octan-3-yliden}-2-fluor-äthan-1-ol 0,3 g der Titelverbindung als farbloses Öl.
IR: 3600, 3420 (breit), 2968, 2932, 2864, 2730, 2225, 1730/cm

Beispiel 44

(5Z)-13,14-Didehydro-1a,1b-dihomo-5-fluor-3-oxa-18,18,19,19-tetradehydro-16,16,20-trimethyl-6a-carba-prostaglandin-$I_2$

In Analogie zu Beispiel 2 erhält man aus 0,3 g des nach Beispiel 43 hergestellten Aldehyds 0,14 g (5Z)-13,14-Didehydro-1a,1b-dihomo-5-fluor-3-oxa-18,18,19,19-tetradehydro-16,16,20-trimethyl-6a-carba-prostaglandin-$I_2$-11,15-diacetat.
Nach Abspaltung der Schutzgruppen erhält man 0,1 g der Titelverbindung als farbloses Öl.
IR: 3605, 3420 (breit), 2970, 2870, 2225, 1720/cm

Beispiel 45

(5E)-(16RS)-1a,1b-Dihomo-16-methyl-3-oxa-18,18,19,19-tetradehydro-6a-carba-prostaglandin-$I_2$-methylester

Zu einer Lösung von 60 mg (5E)-(16RS)-1a,1b-Dihomo-16-methyl-3-oxa-18,18,19,19-tetradehydro-6a-carba-prostaglandin-$I_2$ in 10 ml Dichlormethan tropft man bei 0° C bis zur bleibenden Gelbfärbung eine ätherische Diazomethanlösung. Nach 5 Minuten wird im Vakuum eingedampft und der Rückstand an Kieselgel chromatographiert. Mit Essigester/Hexan (4+1) erhält man 40 mg der Titelverbindung als farbloses Öl.
IR: 3600, 3400 (breit), 2960, 1740, 974/cm

Beispiel 46

(5E)-(16RS)-1 a,1b-Dihomo-16-methyl-3-oxa-18,18,19,19-tetradehydro-6a-carba-prostaglandin-$I_2$-carboxamid

105 mg (5E)-(16RS)-1a,1b-Dihomo-16-methyl-3-oxa-18,18,19,19-tetradehydro-6a-carba-prostaglandin-$I_2$ werden in 3 ml Tetrahydrofuran gelöst und bei 0° C mit 40 mg Triäthylamin und 45 mg Chlorameisensäureisobutylester versetzt. Nach 1 Stunde wird bei 0° C 10 Minuten Ammoniakgas eingeleitet, dann 1 Stunde

bei 24° C belassen. Anschließend wird mit 30 ml Wasser verdünnt, dreimal mit je 30 ml Methylenchlorid extrahiert, die vereinigten organischen Extrakte mit 20 ml Sole geschüttelt, über Magnesiumsulfat getrocknet und im Vakuum eingedampft. Nach Chromatographie des Rückstandes an Kieselgel mit Methylenchlorid/Isopropanol (9 + 1) erhält man 78 mg der Titelverbindung als Öl.
IR: 3610, 3540, 3400 (breit), 2960, 1670, 975/cm

Beispiel 47

(5Z)-(16RS)-1a,1b-Dihomo-5-fluor-16-methyl-3-oxa-18,18,19,19-tetradehydro-6a-carba-prostaglandin-$I_2$-(2,3-dihydroxy-propyl)-amid

195 mg (5Z)-(16RS)-1a,1b-Dihomo-5-fluor-16-methyl-3-oxa-18,18,19,19-tetradehydro-6a-carba-prostaglandin-$I_2$ werden in 5 ml Aceton gelöst und bei 0° C mit 60 mg Triäthylamin und 75 mg Chlorameisensäureisobutylester versetzt. Nach 20 Minuten fügt man eine Lösung von 260 mg 1-Amino-2,3-dihydroxypropan in 8 ml Aceton und 8 ml Acetonitril zu und rührt 2 Stunden bei 20° C. Man engt im Vakuum ein, verdünnt mit Methylenchlorid, schüttelt mit wenig Sole, trocknet die organische Phase mit Magnesiumsulfat und dampft im Vakuum ein. Nach Chromatographie des Rückstandes an Kieselgel erhält man mit Methylenchlorid/Isopropanol (8 + 2) 160 mg der Titelverbindung als farbloses Öl.
IR: 3600, 3400 (breit), 2935, 1645, 974/cm

Beispiel 48

(5Z)-(16RS)-1a,1b-Dihomo-5-fluor-16-methyl-3-oxa-18,18,19,19-tetradehydro-6a-carba-prostaglandin-$I_2$-(4-phenyl)-phenacylester

120 mg (5Z)-(16RS)-1a,1b-Dihomo-5-fluor-16-methyl-3-oxa-18,18,19,19-tetradehydro-6a-carba-prostaglandin-$I_2$ werden in 3 ml Aceton gelöst und mit 90 mg $\omega$-Brom-4-phenylacetophenon und 1 ml Triäthylamin versetzt und über Nacht bei Raumtemperatur gerührt. Man versetzt mit 100 ml Äther, schüttelt zweimal mit je 10 ml Wasser, trocknet über Magnesiumsulfat und dampft im Vakuum ein. Die Reinigung erfolgt durch präparative Dünnschichtchromatographie an Kieselgelplatten, die mit Essigester entwickelt werden. Man erhält 128 mg der Titelverbindung.
IR: 3610, 2940, 1740, 1703, 1602, 974/cm

Beispiel 49

(5E)-(16RS)-1a,1b-Dihomo-16-methyl-3-oxa-18,18,19,19-tetradehydro-6a-carba-prostaglandin-$I_2$-tris-(hydroxymethyl)-aminomethan-salz

Zu einer Lösung von 185 mg (5E)-(16RS)-1a,1b-Dihomo-16-methyl-3-oxa-18,18,19,19-tetradehydro-6a-carba-prostaglandin-$I_2$ in 35 ml Acetonitril fügt man bei 70° C eine Lösung von 60 mg Tris-(hydroxymethyl)-aminomethan in 0,2 ml Wasser. Man läßt unter Rühren abkühlen, dekantiert nach 16 Stunden vom Lösungsmittel und trocknet den Rückstand im Vakuum. Man isoliert 160 mg der Titelverbindung als wachsartige Masse.

**Patentansprüche**

1. Carbacyclinderivate der Formel I,

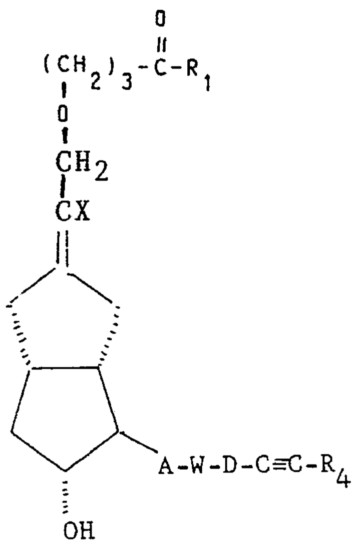

(I),

worin

- $R_1$ Wasserstoff oder Hydroxy,
- X Wasserstoff oder Fluor,
- A eine trans-CH = CH- oder -C≡C-Gruppe,
- W eine Hydroxymethylengruppe, wobei die OH-Gruppe α- oder β-standig sein kann,
- D die Gruppe

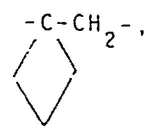

$-CH(CH_3)-CH_2-$ oder $-C(CH_3)_2-CH_2-$,
- $R_4$ Methyl oder Ethyl und,

falls $R_1$ die Bedeutung einer Hydroxygruppe hat, deren Salze mit physiologisch verträglichen Basen bedeuten.

**2.** (5E)-(16RS)-1a, 1b-Dihomo-16-methyl-3-oxa-18,18,19,19-tetradehydro-6a-carba-prostaglandin-$I_2$-methylester

**3.** (5E)-(16RS)-1a, 1b-Dihomo-16-methyl-3-oxa-18,18,19,19-tetradehydro-6a-carba-prostaglandin-$I_2$-carboxamid

**4.** (5Z)-(16RS)-1a, 1b-Dihomo-5-fluor-16-methyl-3-oxa-18,18,19,19-tetradehydro-6a-carba-prostaglandin-$I_2$-(2,3-dihydroxy-propyl)-amid

**5.** (5Z)-(16RS)-1a,1b-Dihomo-5-fluor-16-methyl-3-oxa-18,18,19,19-tetradehydro-6a-carba-prostaglandin-$I_2$-(4-phenyl)-phenacylester

**6.** Verfahren zur Herstellung von Carbacyclin-Derivaten nach Ansprüchen 1-5, dadurch gekennzeichnet, daß man in an sich bekannter Weise eine Verbindung der Formel II

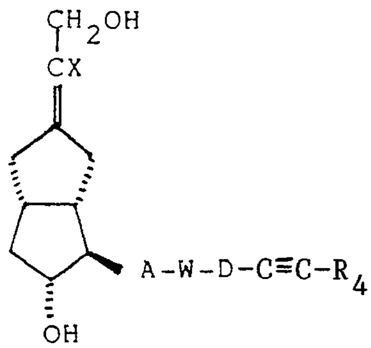

(II),

worin X, $R_4$, A, W und D die in Anspruch 1 angegebenen Bedeutungen aufweisen, gegebenenfalls nach Schutz anwesender freier Hydroxygruppen mit einem Halogenketal der Formel III

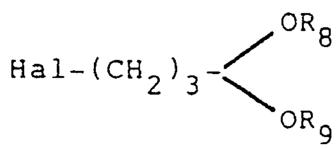

(III),

wobei Hal ein Chlor-, Brom- oder Jodatom, $R_8$ und $R_9$ eine Alkylgruppe mit 1-10 C-Atomen oder $R_8$ und $R_9$ gemeinsam eine ringbildende Gruppe mit 2-10 C-Atomen bedeuten, in Gegenwart einer Base veräthert, mit Säure das Ketal spaltet, geschützte Hydroxygruppen freisetzt und die Aldehydgruppe oxidiert und gegebenenfalls anschließend in beliebiger Reihenfolge Isomere trennt und/oder die resultierende freie Carboxylgruppe verestert und/oder eine veresterte Carboxylgruppe verseift oder eine Carboxylgruppe in ein Amid oder mit einer physiologisch verträglichen Base in ein Salz überführt.

**7.** Arzneimittel, bestehend aus einer oder mehreren Verbindungen der Ansprüche 1-5 und üblichen Hilfs- und Trägerstoffen.

**8.** (5E)-(16RS)-1a,1b-Dihomo-16-methyl-3-oxa-18,18,19,19-tetradehydro-6a-carba-prostaglandin-$I_2$.

**9.** (5E)-(16RS)-13,14-Didehydro-1a,1b-dihomo-16,20-dimethyl-3-oxa-18,18,19,19-tetradehydro-6a-carba-prostaglandin-$I_2$.

**10.** (5Z)-(16RS)-13,14-Didehydro-1a,1b-dihomo-16,20-dimethyl-5-fluor-3-oxa-18,18,19,19-tetradehydro-6a-carba-prostaglandin-$I_2$.

**Claims**

**1.** Carbacyclin derivatives of the formula

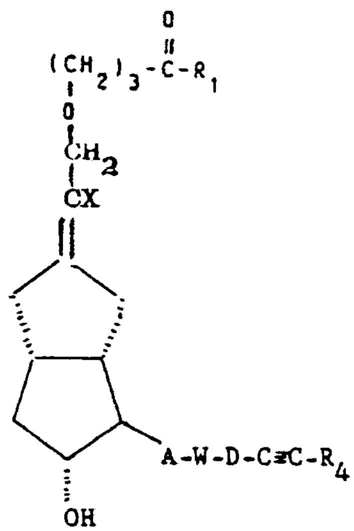

(I)

in which

- $R_1$ represents hydrogen or hydroxy,
- X represents hydrogen or fluorine,
- A represents a trans-CH = CH- or -C≡C- group,
- W represents a hydroxymethylene group wherein the OH group may be in the α- or β-configuration,
- D represents the group

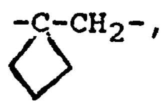

$-CH(CH_3)-CH_2-$ or $-C(CH_3)_2-CH_2-$,

- $R_4$ represents methyl or ethyl and,

if $R_1$ represents a hydroxy group, the salts thereof with physiologically tolerable bases.

**2.** (5E)-(16RS)-1a,1b-Dihomo-16-methyl-3-oxa-18,18,19,19-tetradehydro-6a-carbaprostaglandin-$I_2$-methyl ester.

**3.** (5E)-(16RS)-1a,1b-Dihomo-16-methyl-3-oxa-18,18,19,19-tetradehydro-6a-carbaprostaglandin-$I_2$-carboxamide.

**4.** (5Z)-(16RS)-1a,1b-Dihomo-5-fluoro-16-methyl-3-oxa-18,18,19,19-tetradehydro-6a-carbaprostaglandin-$I_2$-(2,3-dihydroxypropyl)-amide.

**5.** (5Z)-(16RS)-1a,1b-Dihomo-5-fluoro-16-methyl-3-oxa-18,18,19,19-tetradehydro-6a-carbaprostaglandin-$I_2$-(4-phenyl)-phenacyl ester.

**6.** Process for the manufacture of carbacyclin derivatives according to claims 1 to 5, characterised in that, in a manner known per se, a compound of formula II

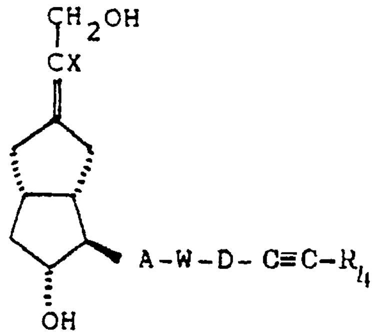

(II),

wherein X, $R_4$, A, W and D have the meanings given in claim 1, is etherified in the presence of a base, optionally after protecting any free hydroxy groups present, with a haloketal of formula III

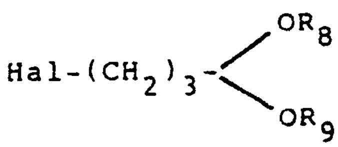

(III)

wherein Hal represents a chlorine, bromine or iodine atom, each of $R_8$ and $R_9$ represents an alkyl group having from 1 to 10 carbon atoms, or $R_8$ and $R_9$ together represent a ring-forming group having from 2 to 10 carbon atoms, the ketal is split with acid, protected hydroxy groups are freed and the aldehyde group is oxidised, and optionally then, in any sequence, isomers are separated and/or the resulting free carboxy group is esterified and/or an esterified carboxy group is hydrolysed or a carboxy group is converted into an amide or is converted into a salt with a physiologically tolerable base.

**7.** Medicament comprising one or more compounds of claims 1 to 5 and customary adjuncts and carriers.

**8.** (5E)-(16RS)-1a,1b-Dihomo-16-methyl-3-oxa-18,18,19,19-tetradehydro-6a-carbaprostaglandin-$I_2$.

**9.** (5E)-(16RS)-13,14-Didehydro-1a,1b-dihomo-16,20-dimethyl-3-oxa-18,18,19,19-tetradehydro-6a-carbaprostaglandin-$I_2$.

**10.** (5Z)-(16RS)-13,14-Didehydro-1a,1b-dihomo-16,20-dimethyl-5-fluoro-3-oxa-18,18,19,19-tetradehydro-6a-carbaprostaglandin-$I_2$.

## Revendications

**1.** Carbacyclines répondant à la formule générale I :

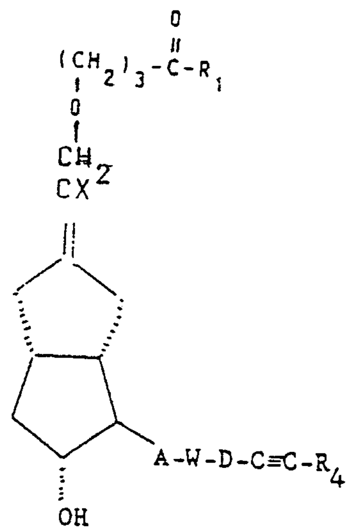

(I)

dans laquelle

$R_1$ représente 1'hydrogène ou un radical hydroxy,

X représente l'hydrogène ou le fluor,

A représente un radical -CH = CH- trans ou un radical -C≡C-,

W représente un radical hydroxyméthylène dont le radical -OH peut avoir la configuration α ou la configuration β,

D représente un radical

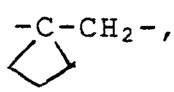

$-CH(CH_3)-CH_2-$ ou $-C(CH_3)_2-CH_2-$, et

$R_4$ représente un radical méthyle ou éthyle,

et également, dans le cas où $R_1$ représente un radical hydroxy, les sels qu'elles forment avec des bases acceptables du point de vue physiologique.

**2.** Ester méthylique de la dihomo-1a,1b méthyl-16 oxa-3 tétradéhydro-18,18,19,19 carba-6a prostaglandine $I_2$ (5E)-(16RS),

**3.** Amide de la dihomo-1a,1b méthyl-16 oxa-3 tétradéhydro-18,18,19,19 carba-6a prostaglandine $I_2$ (5E)-(16RS),

**4.** (Dihydroxy-2,3 propyl)-amide de la dihomo-1a,1b fluoro-5 méthyl-16 oxa-3 tétradéhydro-18,18,19,19 carba-6a prostaglandine $I_2$ (5Z)-(16RS) et

**5.** Ester phényl-4 phénacylique de la dihomo-1a,1b fluoro-5 méthyl-16 oxa-3 tétradéhydro-18,18,19,19 carba-6a prostaglandine $I_2$ (5Z)-(16RS).

**6.** Procédé pour préparer des carbacyclines selon l'une quelconque des revendications 1 à 5, procédé caractérisé en ce que, en opérant de manière connue, on éthérifie en présence d'une base un composé répondant à la formule II :

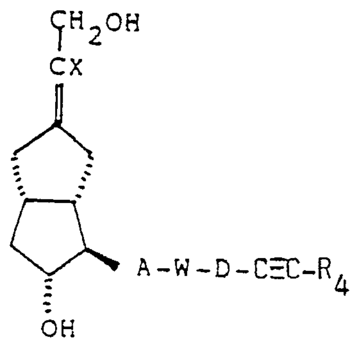

(II)

dans laquelle X, $R_4$, A, W et D ont les significations qui leur ont été données à la revendication 1, - éventuellement après en avoir protégé des radicaux hydroxy libres s'il y en a - avec un halogéno-acétal répondant à la formule générale III :

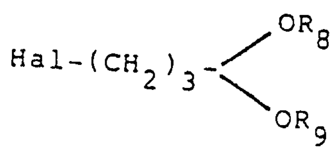

(III)

dans laquelle Hal représente un atome de chlore, de brome ou d'iode, et $R_8$ et $R_9$ représentent chacun un alkyle contenant de 1 à 10 atomes de carbone ou forment ensemble un radical en $C_2$-$C_{10}$ faisant partie d'un cycle,
on coupe l'acétal au moyen d'un acide, on libère des radicaux hydroxy protégés et on oxyde le radical aldéhyde, puis, le cas échéant, en opérant dans l'ordre que l'on veut, on sépare des isomères et/ou on estérifie le radical carboxy libre ainsi formé et/ou on saponifie un radical carboxy estérifié ou on transforme un radical carboxy en un amide ou, au moyen d'une base physiologiquement acceptable, en un sel.

**7.** Médicament constitué d'un ou de plusieurs composés selon l'une quelconque des revendications 1 à 5 et d'adjuvants et excipients usuels.

**8.** Dihomo-1a,1b méthyl-16 oxa-3 tétradéhydro-18,18,19,19 carba-6a prostaglandine $I_2$ (5E)-(16RS).

**9.** Didéhydro-13,14 dihomo-1a,1b diméthyl-16,20 oxa-3 tétradéhydro-18,18,19,19 carba-6a prostaglandine $I_2$ (5E)-(16RS).

**10.** Didéhydro-13,14 dihomo-1a,1b diméthyl-16,20 fluoro-5 oxa-3 tétradéhydro-18,18,19,19 carba-6a prostaglandine $I_2$ (5Z)-(16RS).